Europäisches Patentamt

European Patent Office

Office européen des brevets

(19)

(11) Numéro de publication: **0 319 434 A2**

(12) ## DEMANDE DE BREVET EUROPEEN

(21) Numéro de dépôt: **88420382.9**

(22) Date de dépôt: **16.11.88**

(51) Int. Cl.⁴: **C 07 C 85/26**
**C 07 C 87/14**

(30) Priorité: **30.11.87 FR 8716834**

(43) Date de publication de la demande:
**07.06.89 Bulletin 89/23**

(84) Etats contractants désignés:
**BE DE ES FR GB IT NL**

(71) Demandeur: **RHONE-POULENC CHIMIE**
**25, quai Paul Doumer**
**F-92408 Courbevoie Cédex (FR)**

(72) Inventeur: **Kolischer, Martine**
**11 rue Ferrachat**
**F-69005 Lyon (FR)**

**Vachet, François**
**9 rue Edison**
**F-69150 Decines (FR)**

(74) Mandataire: **Varnière-Grange, Monique et al**
**RHONE-POULENC INTERSERVICES Service Brevets**
**Chimie Centre de Recherches des Carrières B.P. 62**
**F-69192 Saint-Fons Cédex (FR)**

(54) **Procédé de purification de l'hexaméthylènediamine.**

(57) L'invention concerne un procédé de purification de l'hexaméthylènediamine brute cractérisée en ce qu'il comprend : l'introduction du produit à purifier à l'entrée d'un dispositif comportant au moins une zone de cristallisation agitée et munie d'une double enveloppe à travers laquelle circule un fluide réfrigérant et au moins une zone de lavage munie de moyens de chauffage ; le refroidissement contrôlé de la zone de cristallisation pour former une bouillie renfermant au moins 10 % en poids de solide et dont la température est comprise entre 25 et 40,8°C ; l'évacuation d'au moins une partie de cette bouille suivie le cas échéant de sa concentration en solide ; l'introduction de la bouillie résultante en tête de la zone de lavage à contre-courant du liquide de lavage constitué par de l'hexaméthylènediamine purifiée et refondue ; la récupération en pied de ladite zone de lavage l'hexaméthylène-diamine purifiée, sous forme liquide et, l'évacuation d'une portion clarifiée du mélange se trouvant dans la zone de cristallisation.

Bundesdruckerei Berlin

**Description**

## PROCEDE DE PURIFICATION DE L'HEXAMETHYLENEDIAMINE

La présente invention concerne un procédé de purification de l'hexaméthylènediamine (HMD) impure pour l'amener à un haut degré de pureté.

L'hexaméthylènediamine est un produit bien connu, fabriqué à l'échelle industrielle, notamment par hydrogénation catalytique de l'adiponitrile. Elle est principalement utilisée dans la fabrication de polyamides par salification de celle-ci avec une quantité équivalente de diacide carboxylique, suivie de la polycondensation du sel pour former le polyamide.

Bien qu'il existe différents procédés industriels pour fabriquer l'hexaméthylènediamine, aucun d'entre eux ne permet à ce jour d'obtenir directement l'hexaméthylènediamine exempte de produits secondaires et/ou de sous-produits. Il est donc nécessaire de purifier l'hexaméthylènediamine brute obtenue si l'on veut un produit présentant un haut degré de pureté, ce qui est le cas, notamment, lorsque l'hexaméthylènediamine est destinée à la fabrication de polyhexaméthylèneadipamide.

En effet, l'utilisation d'hexaméthylènediamine mal purifiée dans la fabrication de polyhexaméthylèneadipamide provoque un abaissement du poids moléculaire et l'apparition de mauvaises caractéristiques de coloration et d'aptitude à la teinture du polycondensat et affecte diverses autres propriétés physiques et chimiques du polycondensat ou des produits conformés à partir d'un tel polycondensat.

Ainsi, par exemple, l'usage de polycondensat de ce type pour réaliser des filaments ou des fibres conduit à des filaments ou fibres peu résistants et/ou ayant des caractéristiques de coloration qui ne remplissent pas les normes strictes imposées par les industries textiles.

L'hexaméthylènediamine brute, c'est-à-dire issue d'un stade de fabrication peut être classiquement débarrassée des impuretés indésirables en subissant une série de distillations et/ou de traitements chimiques.

Il est cependant bien connu de l'homme de l'art que, même après avoir été soumise à une série de distillations, l'hexaméthylènediamine contient encore une teneur notable en impuretés indésirables. On pense même que ces impuretés se sont formées aussi bien au cours de la fabrication qu'au cours de la purification de l'hexaméthylènediamine. Sans connaître la nature précise de ces impuretés, on pense qu'il s'agit principalement de l'aminohexylidène-imine et de produits de condensation de cette imine, ou d'autres composés présents dans le milieu, avec l'hexaméthylènediamine. Ces impuretés, présentant des liaisons non saturées réduites au polarographe sont dénommées couramment "impuretés réductibles au polarographe".

Par ailleurs, les techniques de distillation et/ou d'extraction dont l'efficacité est insuffisante dans ce cadre particulier présentent en outre au moins l'un des inconvénients suivants :
- elles impliquent une forte consommation énergétique,
- elles présentent une complexité de mise en oeuvre croissante avec les exigences de pureté du produit recherché,
- elles sont un facteur de risque de dégradation du produit recherché aux températures élevées qui peuvent être nécessaires.

Dans la demande de brevet français n° 2341343, il a été indiqué que le degré de pureté de l'hexaméthylènediamine évalué par des mesures de coloration intitulée sans plus ample précision "UV" pouvait passer de 3 800 à 310 par une cristallisation dans des conditions particulières de mise en oeuvre d'un procédé de séparation de substances d'un mélange liquide par cristallisation fractionnée, procédé selon lequel on fait passer plusieurs fois le mélange liquide en écoulement turbulent à travers une zone de cristallisation refroidie indirectement et qui doit être toujours remplie ; on élimine le liquide restant après dépôt d'une couche cristalline sur la paroi de la zone de cristallisation, on lave la surface de la couche cristalline et on la fait fondre ensuite en faisant passer à travers la zone de cristallisation une masse fondue de composition semblable à celle de la couche cristalline, le mélange étant maintenu pendant la cristallisation à la température de cristallisation, la cristallisation étant poursuivie jusqu'à ce que le taux de produit séparé dans la zone de cristallisation par congélation s'élève à 70-98 %.

Selon ce procédé antérieur, il est essentiel que le mélange liquide à cristalliser circule plusiers fois en circuit fermé à travers les zones de cristallisation qui doivent toujours être remplies et que règne un écoulement turbulent dans la zone de cristallisation, la vitesse du mélange liquide étant avantageusement maintenue dans les zones de cristallisation à une valeur comprise entre 0,2 et 6 m/s, la vitesse de croissance au cours de la cristallisation étant avantageusement maintenue aussi constante que possible dans une plage comprise entre 0,05 et 0,5 mm/mn.

L'efficacité d'une telle technique appliquée à la purification de l'hexaméthylènediamine brute ne paraît pas totalement satisfaisante dans la mesure où le gain serait d'un facteur de l'ordre de 12 seulement.

Par ailleurs, la mise en oeuvre d'une telle technique sur une grande quantité de produit à purifier paraît rédhibitoire en raison de l'écoulement turbulent requis qui implique des vitesses de circulation importantes difficiles, voire impossibles, à assurer sur des quantités importantes de produit.

En outre, lorsque le produit à purifier ne comporte pas une teneur très importante en impuretés, sa circulation à grande vitesse dans les tubes constituant la zone de cristallisation cause un blindage pratiquement instantané des parois desdits tubes ; il s'ensuit qu'il est difficile si ce n'est impossible de contrôler la vitesse de croissance ce qui rend pratiquement illusoire une purification supplémentaire dudit produit.

Il a maintenant été trouvé un procédé de purification de l'hexaméthylènediamine brute par cristallisation fractionnée permettant d'obvier aux inconvients précités.

La présente invention a donc pour objet un procédé de purification de l'hexaméthylènediamine brute caractérisé en ce qu'il comprend :

a) l'introduction, de l'hexaméthylènediamine brute présente dans un milieu essentiellement liquide, à l'entrée d'un dispositif comportant au moins une zone de cristallisation agitée et munie d'une double enveloppe à travers laquelle circule un fluide réfrigérant et au moins une zone de lavage munie de moyens de chauffage et distincte de la zone cristallisation ;

b) le refroidissement contrôlé de la zone de cristallisation de telle sorte qu'il se forme une bouillie dont la fraction de solide en suspension représente au moins 10 % en poids et dont la température est comprise entre 25° et 40,8°C ;

c) l'évacuation d'au moins une partie de cette bouillie suivie, le cas échéant, de sa concentration en solide par élimination d'une fraction du liquide qu'elle renferme (ou jus-mère) ;

d) l'introduction de la bouillie résultante en tête de la zone de lavage dans laquelle elle circule à contre-courant du liquide de lavage constitué par une partie de l'hexaméthylènediamine purifiée et refondue ;

e) la récupération en pied de ladite zone de lavage d'hexaméthylènediamine purifiée, sous forme liquide et,

f) l'évacuation d'une portion clarifiée du mélange se trouvant dans la zone de cristallisation.

Par hexaméthylènediamine brute on entend l'hexaméthylènediamine impure à purifier, c'est-à-dire un mélange d'hexaméthylènediamine pure, d'impuretés lourdes et/ou d'impuretés légères dont la nature et les proportions peuvent être très diverses, la teneur totale en impuretés n'excédant préférentiellement pas 5 % en poids dans ledit mélange, voire 3 % en poids.

Par milieu essentiellement liquide, on entend un milieu qui peut résulter de la fusion totale ou partielle de l'hexaméthylènediamine brute, la teneur en solide en suspension étant de préférence inférieure à 1% poids, le cas échéant avec des quantités mineures inférieures à 1% en poids de solvant ou diluant exogène tel que : l'eau et/ou un alcanol en $C_1$-$C_4$.

Il est essentiel dans le cadre du présent procédé que le dispositif comporte au moins une zone de cristallisation agitée et refroidie par circulation d'un fluide réfrigérant dans une double enveloppe.

De manière avantageuse, la cristallisation sera conduite dans une zone de cristallisation également râclée.

Le contrôle du refroidissement peut être assuré par divers moyens bien connus des spécialistes tels que le contrôle de l'écart de température maximal entre le fluide caloporteur circulant dans la double enveloppe de la zone de cristallisation et le milieu constitué par le produit à purifier, la vitesse d'agitation dans la zone de cristallisation, la surface d'échange... de telle sorte que les cristaux apparaissent dans la masse et non sur la paroi. Par ailleurs, une bonne homogénéisation de la température dans le milieu, le maintien en suspension des cristaux et un contact solide-liquide satisfaisant dans la zone de cristallisation par divers moyens connus des spécialistes permettent une mise en oeuvre pratique avantageuse du présent procédé.

Selon la présente invention, il est essentiel que la teneur du solide en suspension dans la bouillie formée dans la zone de cristallisation soit au moins égale à 10 % en poids. Il se révèle avantageux de limiter cette teneur à 50 % en poids au maximum et pour une bonne mise en oeuvre du procédé selon l'invention, cette teneur sera comprise entre 20 et 35 % en poids de ladite bouillie.

La température de refroidissement de la bouillie est comprise entre 25° et 40,8°C.

Sa valeur précise sera avant tout déterminée, au moyen d'essai simple, en fonction du degré de pureté de l'hexaméthylènediamine brute à purifier et de la quantité précise de chacune des impuretés présentes.

L'évacuation d'au moins une partie de cette bouillie peut être réalisée par divers moyens, par exemple, par gravité dans la partie inférieure de la zone de cristallisation et/ou par l'intermédiaire d'une pompe.

Selon la teneur de solide en suspension déjà présente dans la bouillie, il peut se révéler avantageux de faire subir à ladite bouillie une concentration en éliminant, par tout moyen approprié de séparation liquide-solide, une fraction du liquide qu'elle renferme (jus-mère) ; cette élimination étant de manière avantageuse réalisée avec le moins possible d'entraînement de particules solides, par exemple, par recours à un hydrocyclone.

Pour une bonne mise en oeuvre du présent procédé, la concentration ne doit pas amener la teneur en solide au-dessus de 50 % en poids de la bouillie concentrée.

La bouillie résultante, le cas échéant après concentration, est alors introduite en tête d'une zone de lavage dans laquelle elle circule à contre-courant du liquide de lavage constitué par une partie de l'hexaméthylènediamine purifiée et refondue, la zone de lavage étant distincte de la zone de cristallisation et munie de moyens de chauffage.

La zone de lavage se présente le plus souvent sous la forme d'une colonne disposée verticalement et munie en son pied d'un fondoir.

Au démarrage du procédé, les premiers cristaux d'hexaméthylènediamine purifiée, non encore lavés arrivant à proximité du fondoir sont fondus et constituent les premiers volumes du liquide qui sert à laver les cristaux suivants.

Cette colonne présente un gradient de température de haut en bas et permet par les jeux concourants du contre-courant solide-liquide, entre les cristaux qui décantent le long de celle-ci et le liquide provenant de la fusion partielle qui reflue, et de la fusion superficielle des cristaux, de laver la surface des cristaux, d'extraire les inclusions de liquide impur dans les cristaux au fur et à mesure de leur décantation.

En pied de la colonne, on récupère de l'hexaméthylènediamine liquide dont la pureté peut être supérieure à 99,99 %.

Selon une variante avantageuse, une partie du liquide de lavage enrichi en impuretés est recyclée dans la zone de cristallisation ; cette portion représente avantageusement de 30 à 50 % en poids de la production, qui reflue.

Par ailleurs, l'évacuation d'une portion clarifiée du mélange se trouvant dans la zone de cristallisation peut constituer le rejet de l'opération. La clarification est avantageusement conduite dans une zone de clarification située au-dessus de la zone de cristallisation, les deux zones ainsi superposées correspondant en pratique à deux tronçons du même cristallisoir vertical.

Selon une variante avantageuse lorsque la bouillie issue de la zone de cristallisation est soumise à une concentration, les jus-mères issus de cette concentration sont recyclés dans la zone de cristallisation.

Le procédé ainsi décrit peut comporter en outre diverses phases de cristallisations et/ou de recyclages dont le but sera soit d'améliorer le degré de pureté de l'hexaméthylènediamine obtenue en pied de la zone de lavage, soit d'augmenter le rendement de récupération de l'hexaméthylènediamine contenue dans le mélange brut initial, soit d'augmenter simultanément ces deux types de performances.

La présente invention a donc également pour objet un procédé de purification de l'hexaméthylènediamine brute caractérisé en ce qu'il comprend :

a) l'introduction de l'hexaméthylènediamine brute présente dans un milieu essentiellement liquide à l'entrée de la première des zones de cristallisation (zone 1) d'un dispositif comportant au moins 2 zones de cristallisation agitées et munies chacune d'une double enveloppe à travers laquelle circule un fluide réfrigérant, les zones étant disposées en série, le dispositif comportant au moins une zone de lavage munie de moyens de chauffage et distincte de chacune des zones de cristallisation, la seconde zone de cristallisation (zone 2) étant située entre la première zone de cristallisation et la zone de lavage ;

b) le refroidissement contrôlé de la zone 1 de telle sorte qu'il se forme une bouillie dont la fraction de solide en suspension représente au moins 10 % en poids et dont la température est comprise entre 25 et 40,8°C ;

c) l'évacuation d'au moins une partie de cette bouillie suivie, le cas échéant, de sa concentration en solide par élimination d'une fraction du liquide qu'elle renferme (ou jus-mère 1) ;

d) l'introduction de la bouillie résultante (bouillie 1) dans la zone 2 ;

e) le refroidissement contrôlé de la zone 2, de telle sorte qu'il se forme une bouillie dont la fraction de solide en suspension représente au moins 10 % en poids et dont la température est comprise entre 25 et 40,8°C et est supérieure à la température de refroidissement de la zone 1 ;

f) l'évacuation d'au moins une partie de cette bouillie suivie, le cas échéant, de sa concentration en solide par élimination d'une fraction du liquide qu'elle renferme (ou jus-mère 2) ;

g) l'introduction de la bouillie résultante (bouillie 2) en tête de la zone de lavage dans laquelle elle circule à contre-courant du liquide de lavage constitué par une partie de l'hexaméthylènediamine purifiée et refondue ;

h) la récupération en pied de ladite zone de lavage d'hexaméthylènediamine purifiée sous forme liquide et,

i) l'évacuation d'une portion clarifiée du mélange se trouvant dans la zone de cristallisation (zone 1)

Pour une bonne mise en oeuvre de ce procédé comportant au moins deux étages de cristallisation matérialisés par la présence d'au moins deux zones de cristallisation disposées en série, la teneur en solide en suspension dans chacune des bouillie présentes dans les zones de cristallisation représente au maximum 50 % en poids de la bouillie concernée ; cette teneur est avantageusement comprise entre 20 et 35 % en poids. Selon un mode d'exécution avantageux, chacune des zones de cristallisation est râclée.

Dans la série de zones de cristallisation, il est essentiel que la température de chacune des zones de cristallisation se succédant jusqu'à la zone de lavage soit supérieure à celle qui la précède immédiatement dans la série ainsi orientée.

L'écart de température entre deux zones successives sera en général compris entre 0,1 et 3°C.

Lorsqu'une bouillie est issue d'une zone de cristallisation, Elle est introduite, le cas échéant après concentration, dans la zone de cristallisation qui lui succède immédiatement dans la série orientée comme indiqué ci-avant et lorsqu'elle est issue de la zone de cristallisation précédant immédiatement la zone de lavage elle est introduite en tête de ladite zone de lavage. Lorsqu'une bouillie est concentrée avant d'être introduite dans la zone suivante, les jus-mères résultant de la concentration sont alors avantageusement recyclés dans la zone de cristallisation dont est issue la bouillie en cause ; il est également avantageux de recycler au moins une partie du liquide de lavage issu en tête de la zone de lavage dans la zone de cristallisation la précédant immédiatement.

Pour illustrer davantage cet enchaînement des étapes du procédé dans l'hypothèse d'un agencement comportant deux zones de cristallisation en série et une zone de lavage, la zone 1 étant celle à laquelle on alimente l'hexaméthylènediamine brute à purifier, on indique brièvement ce qui suit : la bouillie issue de la zone 1 est introduite, le cas échéant après concentration, dans la zone 2 ; la bouillie issue de la zone 2 est alors introduite, le cas échéant après concentration dans la zone de lavage ; les jus-mères issus de la concentration de la première bouillie mentionnée sont alors recyclés dans la zone de cristallisation 1 et ceux issus de la seconde bouillie mentionnée sont alors recyclés également dans la zone de cristallisation 2 ; au moins une partie du liquide de lavage issu en tête de la zone de lavage est recyclée à la zone de cristallisation 2.

Bien que le nombre de zones de cristallisation disposées en série puisse être grand en théorie, l'économie globale du procédé dictera le nombre à retenir en pratique par un compromis entre ce nombre, la pureté recherchée et le coût de chaque étage. De bons résultats peuvent être obtenus en disposant d'une série de 1 à 5 zones de cristallisation.

Indépendamment du nombre de zones de cristallisation constituant la série précitée, et selon un mode d'exécution avantageux du procédé selon l'invention, ledit procédé comporte en outre :
- l'introduction de la portion clarifiée et évacuée de la zone de cristallisation concernée, zone unique ou zone 1, dans une zone supplémentaire de cristallisation agitée et munie d'une double enveloppe à travers laquelle circule un fluide réfrigérant,
- le refroidissement contrôlé de ladite zone supplémentaire de cristallisation de telle sorte qu'il se forme une bouillie dont la fraction de solide en suspension représente au moins 10 % en poids et dont la température est comprise entre 25 et 38°C et est inférieure à celle de la zone dont est issue la portion clarifiée,
- l'évacuation d'au moins une partie de cette bouillie suivie, le cas échéant de sa concentration en solide par élimination d'une fraction du liquide qu'elle renferme (jus-mères),
- le recyclage de la bouillie résultante dans la zone de cristallisation d'où est issue la portion clarifiée,
- le cas échéant, le recyclage des jus-mères à ladite zone supplémentaire de cristallisation, et
- l'évacuation d'une portion clarifiée du mélange se trouvant dans ladite zone supplémentaire de cristallisation.

Selon ce mode d'exécution, à la série comportant une ou plusieurs zone(s) de cristallisation agencée(s) et fonctionnant comme cela a été décrit précédemment pour aboutir en quelque sorte à la zone de lavage et ainsi, définissant, par convention, un flux orienté de la zone de cristallisation 1 dans laquelle l'hexaméthylènediamine brute à purifier est alimentée vers la zone de lavage dont est issue en pied l'hexaméthylènediamine purifiée recueillie comme production, on adjoint en amont de la zone de cristallisation 1 une zone supplémentaire de cristallisation disposée en série dont l'alimentation comprend une portion clarifiée du mélange se trouvant dans la zone de cristallisation 1 (rejet intermédiaire) ; la bouillie issue de cette zone de cristallisation supplémentaire est alors, le cas échéant après concentration, introduite dans la zone de cristallisation 1, les jus-mères issus le cas échéant de la concentration en cause sont recyclés à la zone de cristallisation supplémentaire et, une portion clarifiée du mélange se trouvant dans ladite zone supplémentaire de cristallisation est évacuée et peut alors constituer le résidu ou rejet final de l'opération.

Cette zone de cristallisation supplémentaire traitant en quelque sorte le rejet intermédiaire peut bien entendu comporter une pluralité de cristallisoirs disposés en série et fonctionnant de manière analogue à celle décrite pour la partie de l'installation et du procédé destinée à la production.

Bien entendu, diverses variantes ou modifications peuvent être apportées au procédé qui vient d'être décrit qui apparaîtront immédiatement à l'homme de métier. Ainsi, par exemple, la zone de lavage qui est le plus souvent constituée par une colonne de lavage verticalement disposée peut être constituée, par exemple, par au moins deux dispositifs de ce type disposés en parallèle. De même, une ou plusieurs zones de cristallisation situées entre la zone de cristallisation 1 et la zone de lavage pourront comporter une zone de clarification analogue à celles dont il a été question dans le corps du présent mémoire ; une portion clarifiée du mélange se trouvant dans la zone en cause pourra alors être introduite dans la zone de cristallisation située immédiatement en amont au sens de la convention choisie et explicitée ci-avant.

En outre, l'alimentation en hexaméthylènediamine brute à purifier peut être réalisée de diverses manières selon le degré de pureté initial du mélange brut. Ainsi lorsque le mélange brut atteint un degré de pureté compris entre 99,3 et 99,8 %, par exemple, il peut être alimenté dans le cristallisoir précédant immédiatement la zone de lavage ; lorsque le mélange brut atteint un degré de pureté compris entre 99,1 et 99,3 % il pourra avantageusement être alimenté dans le cristallisoir précédant celui situé immédiatement avant la zone de lavage. L'alimentation peut également être répartie dans les divers cristallisoirs. Comme cela apparaîtra immédiatement à l'homme de l'art, lorsque le degré de pureté du mélange brut à purifier augmente, le nombre d'étages de purification nécéssaires diminue pour ne requérir la mise en oeuvre que d'un seul cristallisoir situé avant la zone de lavage d'où est évacuée la production ; par contre, la quantité de flux résiduel pour une production donnée augmente et il faut alors, pour une bonne économie du procédé, traiter le rejet par la mise en oeuvre d'1 à 5 cristallisoirs en série dans la partie amont, au sens de la convention prise ci-avant.

Par ailleurs, et pour une bonne mise en oeuvre du présent procédé on peut disposer immédiatement en aval du cristallisoir traitant un rejet de l'opération, au sens indiqué ci-avant, ou du cristallisoir dont est issu le résidu au sens également précisé ci-avant, ce cristallisoir étant celui dont la température de fonctionnement est la plus basse dans l'opération, une zone supplémentaire de lavage munie de moyens de chauffage. Cette zone est le plus souvent sous forme d'une colonne verticale munie en son pied d'un fondoir. La bouillie issue du cristallisoir incriminé et qui, le plus souvent est celui qui fonctionne à la température la plus basse de l'opération est alimentée, le cas échéant après concentration, en tête de la dite zone supplémentaire de lavage ou celle circule à contre-courant du liquide de lavage constitué par une portion de l'hexaméthylènediamine purifiée (mais encore relativement impure) refondue ; une autre portion de l'hexaméthylènediamine purifiée quoiqu'encore relativement impure est prélevée en pied de cette zone de lavage pour être recyclée dans le cristallisoir situé immédiatement en aval selon la convention précitée, c'est-à-dire dans celui des cristallisoirs dont la température de fonctionnement est supérieure à celle du cristallisoir d'où est issue la bouillie à laver tout en étant la plus proche de celle-ci.

Bien entendu les jus-mères issus de la phase de concentration précédant, le cas échéant, l'introduction dans la zone de lavage supplémentaire, peuvent avantageusement être recyclés au cristallisoir dont est issue la bouillie en cause.

La teneur en solide du flux alimenté dans la zone de lavage supplémentaire est de 10 à 50 % en poids.

De manière avantageuse, une portion de liquide de lavage est recyclé au cristallisoir d'où est issue la bouillie en cause.

La zone de lavage supplémentaire est avantageusement située en aval du cristallisoir dont est issu le résidu de l'opération.

Comme cela apparaîtra à la lecture du présent mémoire, le présent procédé dans l'un de ses modes d'exécution avantageux peut être qualifié de modulaire en ce sens qu'il comporte en quelque sorte, d'une part une zone de cristallisation en purification et une zone de lavage de la production et d'autre part une zone de cristallisation en enrichissement et, le cas échéant, une zone de lavage supplémentaire permettant d'évacuer le résidu de l'opération, chaque zone de cristallisation pouvant être constituée par de 1 à 5 cristallisoirs montés en série, l'alimentation en hexaméthylènediamine brute à purifier pouvant être répartie sur plusieurs cristallisoirs de la zone travaillant en purification ou bien réalisée sur un seul des cristallisoirs de cette zone, voire sur l'unique cristallisoir de cette zone.

Des résultats satisfaisants peuvent être obtenus par la mise en oeuvre du procédé selon l'invention sur une installation comportant en série au maximum 5 cristallisoirs voire, au maximum 4.

Compte-tenu, d'une part des appareils disponibles sur le marché et, d'autre part de la qualité de l'échange thermique de chaque appareil, qui est fonction de la surface d'échange pour un volume donné d'appareil pour chaque cristallisoir des zones de cristallisation en purification ou en enrichissement, on évacue de 10 à 50 parties en poids par heure de portion clarifiée du mélange se trouvant dans un cristallisoir pour de 90 à 50 parties en poids de bouillie qui en sort par heure. De préférence, ces flux sont respectivement de 60 à 80 parties en poids par heure de bouillie pour de 40 à 20 parties en poids par heure de portion clarifiée.

Les exemples ci-après illustrent l'invention.

Exemple 1 :

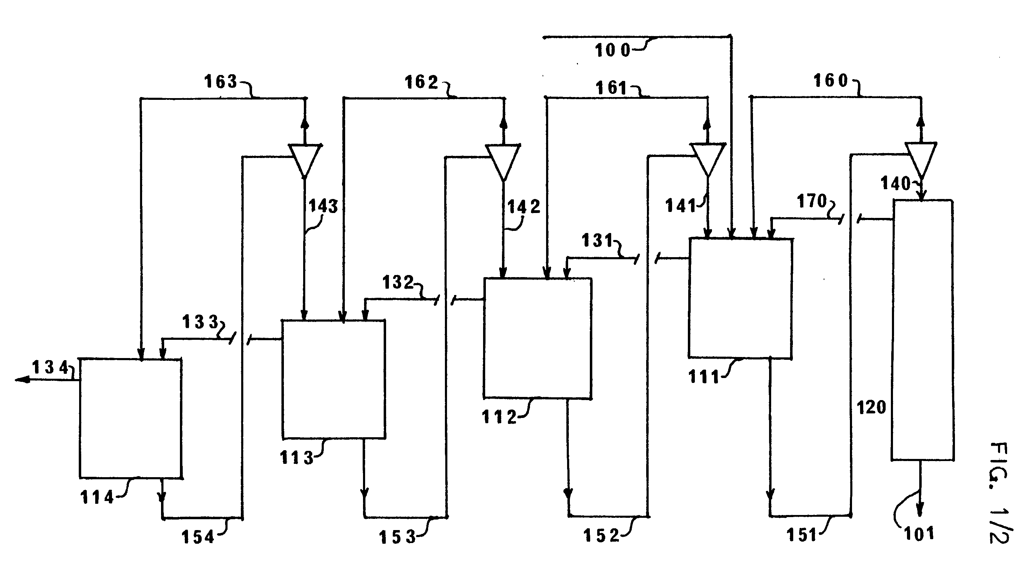

L'installation utilisée comprend deux parties, une partie appelée par convention -zone de cristallisation en purification- et, une partie appelée par convention -zone de traitement du rejet primaire ou de cristallisation en enrichissement-, l'ensemble est représenté schématiquement par la figure 1 annexée.

La zone de cristallisation en purification comporte, d'une part, un cristallisoir (111) agité et râclé, muni d'une double enveloppe (non représentée) à travers laquelle circule un fluide caloporteur et, d'autre part, une tour de lavage (120) munie en son pied d'un fondoir non représenté. Le cristallisoir (111) est pourvu notamment d'un conduit (100) par lequel on introduit la mélange d'HMD brut à purifier et d'un conduit (151) pour évacuer une portion de la bouillie de cristaux et l'introduire dans un hydrocyclone (schématisé par un triangle dont une pointe est dirigée vers le bas de la figure 1). La colonne (120) est pourvue d'un conduit (140) d'alimentation situé en sa tête, d'un conduit (101) d'évacuation de la production et d'un conduit (170) permettant de recycler une portion du liquide de lavage au cristallisoir (111).

La zone de traitement du rejet primaire comporte 3 cristallisoirs agités et râclés, munis chacun d'une double enveloppe à travers laquelle circule un fluide caloporteur et montés en série (112), (113) et (114).

Le cristallisoir (112) est monté en série avec le cristallisoir (111) ; une partie de l'alimentation du cristallisoir (112) est une portion clarifiée du mélange se trouvant dans le cristallisoir (111) qui est introduite dans le cristallisoir (112) par le conduit (131).

Le résidu (ou rejet final de l'opération) évacué par le conduit (134) est une portion clarifiée du mélange se trouvant dans le cristallisoir (114).

Les températures respectives au sein de chacun des cristallisoirs sont les suivants :

(111) : 41 , 2 °C
(112) : 39 , 9 °C
(113) : 39 , 6 °C
(114) : 35 , 0 °C

Par le conduit (100) on introduit en tête du cristallisoir (111) 4200 parties (en poids) par heure d'un mélange liquide contenant 99,495 % (en poids) d'HMD et 0,505 % en poids d'impuretés. La teneur en diaminocyclohexane (DCH), impureté choisie comme traceur est de 0,20 % (poids). Le mélange alimenté est à la température minimale de 41,8°C.

On produit en pied de la tour (120) 4170 parties (poids) par heure d'HMD à 99,999 % en poids ; la teneur en impuretés est de 10 ppm et celle en DCH de 3 ppm.

On produit en résidu, rejet final évacué par le conduit (134), de 30 parties (poids) par heure, qui renferme 30 % en poids d'HMD et 70 % en poids d'impuretés. La teneur en DCH dudit rejet final est de 28 % en poids.

En pied de chacun des cristallisoirs (111 à 114) sont produites des bouillies respectivement évacuées par les conduits (151 à 154) et qui contiennent environ 25 % en poids de solide pour 75 % en poids de liquide ; les flux respectifs et la teneur globale en impuretés sont indiqués dans le tableau I.1 ci-après :

TABLEAU I.1

| bouillies | parties/heure | impuretés (% poids) |
|---|---|---|
| (151) | 31 200 | 1,41 |
| (152) | 21 550 | 2,10 |
| (153) | 10 750 | 2,82 |
| (154) | 5 400 | 12,00 |

Chacune de ces bouillies est concentrée pour former, d'une part, un flux essentiellement liquide (renfermant environ 2 % en poids de particules solides) et d'autre part un flux dont la teneur en particules solides atteint 45 % en poids ; ainsi, en se référant au cristallisoir (112), par exemple, la bouillie (152) donne naissance à un flux (141) concentré en solide et à un flux (161) de liquide à faible teneur en solide. Chaque flux liquide (160), (161), (162) et (163) est respectivement recyclé au cristallisoir (111), (112), (113) et (114) d'où sont respectivement issues les bouillies (151), (152), (153) et (154) ; les bouillies concentrées (141) à (143) sont alimentées respectivement en tête des cristallisoirs (111) à (113).

En tête de chacun des 4 cristallisoirs (111) à (114) sont prélevées des portions clarifiées respectivement par les conduits (131) à (134) ;
la portion (131) est introduite en tête du cristallisoir (112) ;
la portion (132) est introduite en tête du cristallisoir (113) ;
la portion (133) est introduite en tête du cristallisoir (114) ;
la portion (134) constitue le résidu de l'opération dont il a déjà été question.

Les flux de ces divers fractions ainsi que leur teneur en impuretés sont indiqués dans le tableau I.2 ci-après :

TABLEAU I.2.

| fraction | parties/heure | impuretés (% poids) |
|---|---|---|
| (131) | 11 518 | 1,39 |
| (132) | 5 784 | 2,05 |
| (133) | 1 175 | 2,89 |
| (134) | 160 | 15,00 |

Exemple 2 :

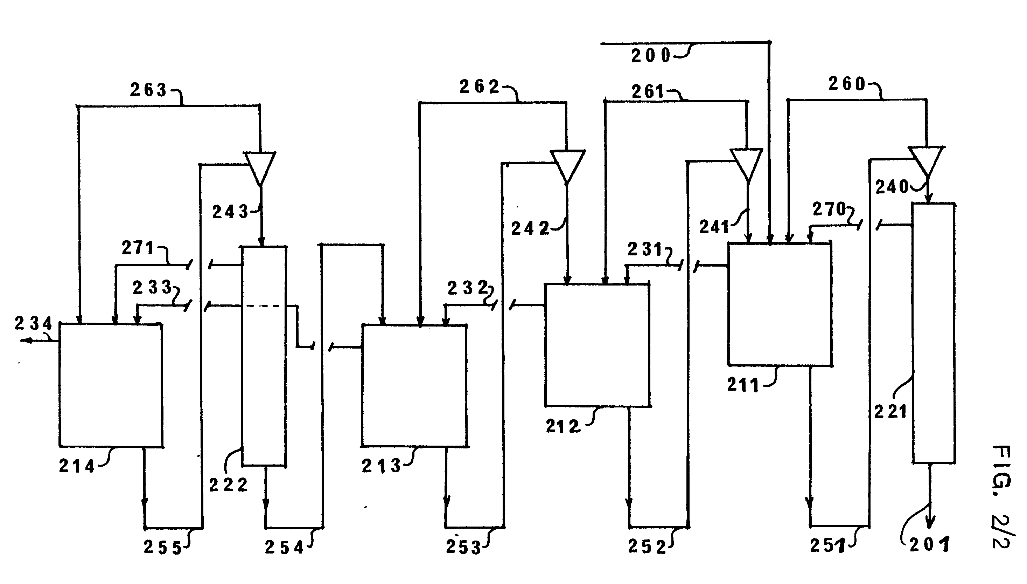

L'installation utilisée comprend deux parties, une partie appelée par convention -zone de cristallisation en purification- et, une partie appelée par convention- zone de traitement du rejet primaire ou de cristallisation en enrichissement-, l'ensemble est représenté schématiquement par la figure 2 annexée.

La zone de cristallisation et de purification comporte, d'une part, un cristallisoir (211) agité et râclé, muni d'une double enveloppe (non représentée) à travers laquelle circule un fluide caloporteur et, d'autre part, une tour de lavage (221) muni en son pied d'un fondoir non représenté. Le cristallisoir (211) est pourvu notamment d'un conduit (200) par lequel on introduit le mélange d'HMD brut à purifier et d'un conduit (251) pour évacuer une portion de la bouillie de cristaux et l'introduire dans un hydrocyclone (schématisé par un triangle dont une pointe est dirigée vers le bas de la figure 2).

La colonne (221) est pourvue d'un conduit (240) d'alimentation situé en sa tête, d'un conduit (201) d'évacuation de la production et d'un conduit (270) permettant de recycler une portion de liquide de lavage au cristallisoir (211).

La zone de traitement du rejet primaire comporte d'une part, 3 cristallisoirs agités et râclés, munis chacun d'une double enveloppe à travers laquelle circule un fluide caloporteur et montés en série (212), (213) et (214) et d'autre part, une tour de lavage (222).

Le cristallisoir (212) est monté en série avec le cristallisoir (211) ; une partie de l'alimentation du cristallisoir (212) est une portion clarifiée de mélange se trouvant dans le cristallisoir (211) qui est introduite dans le cristallisoir (212) par le conduit (231).

Le résidu (ou rejet final de l'opération) évacué par le conduit (234) est une portion clarifiée du mélange se trouvant dans le cristallisoir (214).

La tour de lavage (222) est munie en son pied d'un fondoir (non représenté) et est alimentée en sa tête au moyen du conduit (243) par une fraction enrichie en solide de la bouillie issue du cristallisoir (214) par le conduit (255) qui introduit la bouillie en cause dans un hydrocyclone (représenté par un triangle pointe en bas) ; la fraction est introduite dans la tour de lavage à contre-courant du liquide de lavage résultant de la fusion opérée en pied de ladite tour ; une portion dudit liquide est prélevée en pied de la tour par le conduit (254) pour être recyclée en tête du cristallisoir (213). Une autre portion dudit liquide est prélevée en tête de la tour par le conduit (271) pour être recyclée en tête du cristallisoir (214).

Les températures respectives au sein de chacun des cristallisoirs sont les suivantes :
(211) : 40,21° C
(212) : 39,90° C
(213) : 39,60° C
(214) : 32,60° C

Par le conduit (200) on introduit en tête du cristallisoir (211) 4330 parties par heure d'un mélange liquide contenant 99,3 % en poids d'HMD et 0,7 % en poids d'impuretés. La teneur du mélange en DCH (impureté choisie comme traceur) est de 0,20 % en poids dans ce mélange ; le mélange est alimenté à la température de 50° C. On produit en pied de la tour (221) 4170 parties par heure d'HMD à 99,998 % en poids, la teneur en impuretés étant de 10 ppm, celle en DCH de 3 ppm, cette production étant évacuée par le conduit (201). On produit un résidu, rejet final évacué par le conduit (234), de 160 parties par heure qui renferme 81 % en poids d'HMD et 19 % en poids d'impuretés. La teneur en DCH dudit rejet final est de 5,3 % en poids.

En pied de chacun des cristallisoirs (211 à 214) sont produites des bouillies respectivement évacuées par les conduits (251), (252), (253) et (255) qui contiennent environ 25 % en poids de solide pour 75 % en poids de liquide, les flux respectifs et la teneur globale en impuretés étant indiqués dans le tableau II.1 ci-après.

## TABLEAU II.1

| Bouillies | parties/heure | impuretés (% poids) |
|---|---|---|
| 251 | 31 200 | 1,41 |
| 252 | 21 550 | 2,10 |
| 253 | 10 750 | 2,82 |
| 255 | 5 400 | 19,00 |

Chacune de ces bouillies est concentrée pour former, d'une part, un flux essentiellement liquide (renfermant environ 2 % en poids de particules solides) et d'autre part un flux dont la teneur en particules solides atteint 45% en poids ; ainsi, en se référant au cristallisoir (212), par exemple, la bouillie (252) donne naissance à un flux (241) concentré en solide et à un flux (261) de liquide à faible teneur en solide. Chaque flux liquide (260), (261), (262) et (263) est respectivement recyclé au cristallisoir (211), (212), (213) et (214) d'où sont respectivement issues les bouillies (251), (252), (253) et (255) ; les bouillies concentrées (241) et (242) sont alimentées respectivement en tête des cristallisoirs (211) et (212).

En tête de chacun des cristallisoirs (211) à (214) sont prélevées des portions clarifiées respectivement par les conduits (231) à (234) ;
la fraction (231) est introduite en tête du cristallisoir (212) ;
la fraction (232) est introduite en tête du cristallisoir (213) ;
la fraction (233) est introduite en tête du cristallisoir (214) ;
la fraction (234) constitue le rejet principal dont il a déjà été question.

Les flux de ces diverses fractions ainsi que leur teneur en impuretés sont indiqués dans le tableau II.2 ci-après.

## TABLEAU II.2

| fraction | parties/heure | impuretés (% poids) |
|---|---|---|
| (231) | 11 518 | 1,39 |
| (232) | 5 784 | 2,05 |
| (233) | 1 175 | 2,89 |
| (234) | 160 | 19,00 |

## Revendications

1. - Procédé de purification de l'hexaméthylènediamine brute caractérisé en ce que il comprend :
a) l'introduction de l'hexaméthylènediamine brute présente dans un milieu essentiellement liquide, à l'entrée d'un dispositif comportant au moins une zone de cristallisation agitée et munie d'une double enveloppe à travers laquelle circule un fluide réfrigérant et au moins une zone de lavage munie de moyens de chauffage et distincte de la zone cristallisation ;
b) le refroidissement contrôlé de la zone de cristallisaiton de telle sorte qu'il se forme une bouillie dont la fraction de solide en suspension représente au moins 10 % en poids et dont la température est comprise entre 25° et 40,8°C ;
c) l'évacuation d'au moins une partie de cette bouillie suivie, le cas échéant, de sa concentration en solide par élimination d'une fraction du liquide qu'elle renferme (ou jus-mère) ;
d) l'introduction de la bouillie résultante en tête de la zone de lavage dans laquelle elle circule à contre-courant du liquide de lavage constitué par une partie de l'hexaméthylènediamine purifiée et refondue ;
e) la récupération en pied de ladite zone de lavage d'hexaméthylènediamine purifiée, sous forme liquide et,
f) l'évacuation d'une portion clarifiée du mélange se trouvant dans la zone de cristallisation.

2. - Procédé selon la revendication 1, caractérisé en ce que la teneur en solide en suspension dans la bouillie présente dans la zone de cristallisation représente au maximum 50 % en poids de ladite bouillie.

3. - Procédé selon la revendication 1, caractérisé en ce que la teneur en solide en suspension dans la bouillie présente dans la zone de cristallisation représente entre 20 et 35% en poids de ladite bouillie.

4. - Procédé selon la revendication 1, caractérisé en ce qu'il comprend en outre le recyclage des jus-mères issus de la phase de concentration à la zone de cristallisation.

5. - Procédé selon la revendication 1, caractérisé en ce qu'il comprend en outre le recyclage d'au moins une partie du liquide de lavage issu en tête de la zone de lavage vers la zone de cristallisation.

6. - Procédé de purification de l'hexaméthylènediamine brute caractérisé en ce qu'il comprend :
a) l'introduction de l'hexaméthylènediamine brute présente dans un milieu essentiellement liquide à l'entrée de la première des zones de cristallisation (zone 1) d'un dispositif comportant au moins 2 zones de cristallisation agitées et munies chacune d'une double enveloppe à travers laquelle circule un fluide réfrigérant, les zones étant disposées en série, le dispositif comportant au moins une zone de lavage munie de moyens de chauffage et distincte de chacune des zones de cristallisation, la seconde zone de cristallisation (zone 2) étant située entre la première zone de cristallisation et la zone de lavage ;
b) le refroidissement contrôlé de la zone 1 de telle sorte qu'il se forme une bouillie dont la fraction de solide en suspension représente au moins 10 % en poids et dont la température est comprise entre 25 et 40,8°C ;

c) l'évacuation d'au moins une partie de cette bouillie suivie, le cas échéant, de sa concentration en solide par élimination d'une fraction du liquide qu'elle renferme (ou jus-mère) ;

d) l'introduction de la bouillie résultante (bouillie 1) dans la zone 2 ;

e) le refroidissement contrôlé de la zone 2 de telle sorte qu'il se forme une bouillie dont la fraction de solide en suspension représente au moins 10 % en poids et dont la température est comprise entre 25 et 40,8°C, et est supérieure à la température de refroidissement de la zone 1 ;

f) l'évacuation d'au moins une partie de cette bouillie suvie, le cas échéant, de sa concentration en solide par élimination d'une fraction du liquide qu'elle renferme (ou jus-mère 2) ;

g) l'introduction de la bouillie résultante (bouillie 2) en tête de la zone de lavage dans laquelle elle circule à contre-courant du liquide de lavage constitué par une partie de l'hexaméthylènediamine purifiée et refondue ;

h) la récupération en pied de ladite zone de lavage d'hexaméthylènediamine purifiée sous forme liquide, et

i) l'évacuation d'une portion clarifiée du mélange se trouvant dans la zone 1.

7. - Procédé selon la revendication 6, caractérisé en ce que la teneur en solide en suspension dans chacune des bouillies présentes dans les zones de cristallisation représente au maximum 50 % en poids de ladite bouillie.

8. - Procédé selon la revendication 6, caractérisé en ce que la teneur en solide en suspension dans chacune des bouillies présentes dans les zones de cristallisation représente de 20 à 35 % en poids.

9. - Procédé selon l'une quelconque des revendications 6 à 8 caractérisé en ce qu'il comporte en outre les recyclages des jus-mères 1 et des jus-mères 2 respectivement dans les zones de cristallisation 1 et 2 et le recyclage d'au moins une partie du liquide de lavage issu en tête de la zone de lavage dans la zone de cristallisation 2.

10. - Procédé selon l'une quelconque des revendications 6 à 8 caractérisé en ce que l'écart de température entre les 2 zones de cristallisation est compris entre 0,1 et 3°C.

11. - Procédé selon l'une quelconque des revendications précédentes caractérisé en ce qu'il comporte, en outre :- l'introduction de la portion clarifiée et évacuée de la zone de cristallisation concernée, dans une zone supplémentaire de cristallisation agitée et munie d'une double enveloppe à travers laquelle circule un fluide réfrigérant,

- le refroidissement contrôlé de ladite zone supplémentaire de cristallisation de telle sorte qu'il se forme une bouillie dont la fraction de solide en suspension représente au moins 10 % en poids et dont la température est comprise entre 25 et 38°C et est inférieure à celle de la zone dont est issue la portion clarifiée,

- l'évacuation d'au moins une partie de cette bouillie suivie, le cas échéant de sa concentration en solide par élimination d'une fraction du liquide qu'elle renferme (jus-mères),

- le recyclage de la bouillie résultante dans la zone de cristallisation d'où est issue la portion clarifiée.

- le cas échéant, le recyclage des jus-mères à ladite zone supplémentaire de cristallisation, et

- l'évacuation d'une portion clarifiée du mélange se trouvant dans ladite zone supplémentaire de cristallisation, comme résidu ou rejet final de l'opération.

12. - Procédé selon la revendication 11, caractérisé en ce qu'il comporte en outre le traitement de la bouillie issue du cristallisoir produisant un rejet ou le résidu de l'opération, cette bouillie étant le cas échéant clarifiée, réalisé par :

- l'introduction en tête d'une zone de lavage supplémentaire, de la bouillie résultante où elle circule à contre-courant du liquide de lavage constitué par une portion de l'hexaméthylènediamine purifiée quoiqu'encore relativement impure refondue,

- le prélèvement en pied de ladite zone de lavage d'une portion de l'hexaméthylènediamine purifiée quoiqu'encore relativement impure pour la recycler au cristallisoir dont la température de fonctionnement est supérieure à celle du cristallisoir d'où est issue la bouillie en cause tout en étant la plus proche de celle-ci,

- le cas échéant, le recyclage des jus-mères issus de la phase de concentration, au cristallisoir d'où est issue la bouillie en cause, et

- le cas échéant, le recyclage d'une portion du liquide de lavage, au cristallisoir d'où est issue la bouillie.

FIG. 1/2

FIG. 2/2